# EUROPEAN PATENT SPECIFICATION

(11) **EP 3 289 955 B1**
(45) Date of publication and mention of the grant of the patent: **26.08.2020**
(21) Application number: 16785811.7
(22) Date of filing: 31.03.2016
(51) Int. Cl.: A61B 1/00

(54) **HARD-TUBE ENDOSCOPE**
HARTROHRENDOSKOP
ENDOSCOPE À TUBE DUR

(30) Priority: 29.04.2015 CN 201520271895 U
(43) Date of publication of application: 07.03.2018
(73) Proprietor: Shanghai Anqing Medical Instrument Co. Ltd., Shanghai 201201 (CN)
(72) Inventor: ZHOU, Zhenhua, Shanghai 201201 (CN); YUAN, Zheng, Shanghai 201201 (CN)
(74) Representative: Schäfer, Matthias W.
(86) International application number: PCT/CN2016/078125
(87) International publication number: WO 2016/173380

(56) References cited:
- EP-A2- 0 669 105
- CN-A- 101 933 795
- CN-A- 104 039 212
- CN-U- 204 654 864
- JP-B2- 5 006 706
- US-A- 5 549 637
- US-A1- 2009 281 388

## Description

### BACKGROUND OF THE INVENTION

### 1. Field of the Invention

The present application relates to the field of medical instruments, and in particular to a hard-tube endoscope.

### 2. Description of Related Art

As one kind of endoscopes, hard-tube endoscopes are a medical instrument which is widely applied to internal inspection on a human body at present, and the hard-tube endoscopes allow inspection to be performed safely and conveniently in a clinic without the need of hospitalization.

General hard-tube endoscopes can perform inspection on a patient by means of an eyepiece or an electronic camera, and visual inspection often causes missed diagnosis, misdiagnose etc. Besides, due to the fact that insertion tubes of the hard-tube endoscopes in the prior art are usually straight tubes, a patient can only be observed from one direction, and the observation range is small.

For example, China invention patent application CN103391741 discloses an endoscope, according to which an LED light source unit is disposed in a front hard portion of a front end portion forming an insertion portion of the endoscope, wherein the LED light source unit is provided with a ceramic substrate for an LED; an LED light source, installed on the front-end side of the ceramic substrate for the LED; an LED cable, inserted into the insertion portion of the endoscope in a penetrating mode and led to the front end portion to be connected with a conduction pattern of the ceramic substrate for the LED; and a reinforcing component, integrally fixed to the ceramic substrate for the LED, and covering a ceramic notch portion configured to expose the conduction pattern of the ceramic substrate for the LED.

For another example, China invention patent application CN101267762 discloses an endoscope, and the endoscope is provided with a long and thin insertion portion inserted into a body cavity, and a hard base portion disposed on the base-end side of the insertion portion (22). Transmission lines including an optical fibre and a CCD cable are disposed from the interior of the insertion portion to the base portion in a penetrating mode. A retaining portion is disposed inside the base portion, and the retaining portion retains the transmission lines in the way of limiting the movement state of the transmission lines to be orthogonal to the axial direction of the insertion portion and the base portion. A connecting device portion is disposed on the base portion, and the connecting device portion is used for connecting the ends of the transmission lines which penetrate through the retaining portion to extend out and are connected to an external device.

US 5 549 637 A discloses an articulated medical instrument having a substantially elongated body member divided into a plurality of segments and a tool head receiving means.

CN 104 039 212 A discloses a further insertion instrument, in particular an endoscope, having an elongated insertion portion and an action portion.

EP 0 669 105 A2 discloses a further surgical instrument including a rigid member with a bend region carrying a surgical tool at its distal end.

According to the endoscopes described above, even though the light source is disposed inside, or the volume of the endoscope is reduced, the range of patient observation is still small since the insertion tube of the endoscope is a straight tube; in order to enlarge the observation range, a user needs to rotate the endoscope or insert the endoscope into an inspected area again, which only increases the pain of a patient.

Therefore, comprehensive inspection of a patient cannot be achieved with the hard-tube endoscopes in the prior art due to the small vision field, and missed diagnosis and misdiagnose are caused easily, or harm can be done to a patient due to repeated puncture.

### SUMMARY OF THE APPLICATION

The present application is directed to provide a hard-tube endoscope, comprising an insertion tube, a camera, a light source and a handle. The camera and the light source are disposed on an end face of an insertion end of the insertion tube, and a non-insertion end of the insertion tube is connected to the handle. The hard-tube endoscope has an insertion portion that further comprises a bending portion. The bending portion is located at a part, locatable inside a human body, on the insertion tube, and a control portion configured to control the bending portion to be bent is disposed on the handle.

The control portion comprises an inner ring sleeve and an outer ring sleeve, and the outer ring sleeve is engaged with the inner ring sleeve by means of threads. The outer ring sleeve can rotate only around the axis of the handle with respect to the handle, and the inner ring can only axially move with respect to the axis of the handle.

The insertion tube comprises an elastic inner tube and an elastic outer tube sheathed by each other, and the inner tube and the outer tube are fixedly connected at the position of the insertion end. A non-insertion end of the inner tube in the handle is fixedly connected to the inner ring sleeve, and a non-insertion end of the outer tube is fixedly connected to the handle.

When the outer ring sleeve is rotated and the inner ring sleeve axially moves along the upstream side of the insertion direction along with the rotation of the outer ring sleeve, the inner ring sleeve drives the inner tube to axially move along the upstream side of the insertion direction, and as the movement distance of the inner tube increases gradually, the bending degree of the bending portion also increases gradually.

When the outer ring sleeve is rotated reversely, the inner ring sleeve axially moves along the downstream side of the insertion direction along with the rotation of the outer ring sleeve, so as to drive the inner tube to axially move along the downstream side of the insertion direction. As the movement distance of the inner tube increases gradually, the bending degree of the bending portion reduces gradually till bending disappears.

Preferably, the bending range of the bending portion is 0° to 80°.

Further, the bending portion comprises a plurality of annular slots formed in outer wall faces of the outer tube and the inner tube.

Preferably, the bending portion is further located at a starting site of the insertion end of the insertion tube.

Further, the annular slots are parallel to one another. The annular slots are perpendicular to the axis of the insertion tube.

Preferably, the outer tube is further sheathed by a protection tube, one end of the protection tube is fixedly connected with the insertion end of the insertion tube, and the other end of the protection tube is disposed in the handle in a floating mode.

Further, the protection tube is a hard tube, and a plurality of annular slots are also formed in the position, corresponding to the bending portion of the insertion tube, of an outer wall face of the protection tube.

Preferably, the protection tube is a flexible tube.

Further, an instrument tube is arranged in the inner tube of the insertion tube. An instrument outlet of the instrument tube is formed in an end face of the insertion end of the insertion tube, and an instrument inlet of the instrument tube is formed in the handle or in peripheral walls of the insertion tube the protection tube.

Preferably, a water tube is disposed in the insertion tube, a water outlet of the water tube is formed in the end face of the insertion end of the insertion tube, and a water inlet of the water tube is formed in the handle or in peripheral walls of the insertion tube and the protection tube.

Based on the above, the hard-tube endoscope according to the present application allows the insertion end of the insertion tube to be bent as needed, so as to obtain a larger vision field.

In order to make the aforementioned content of the present application comprehensible, embodiments accompanied with figures are described in detail below.

### BRIEF DESCRIPTION OF THE DRAWINGS

The present application will be described by referring to drawings hereinafter.
Fig. 1 is an overall structure diagram of a hard-tube endoscope according to the present application.
Fig. 2 is a cross-sectional view of Fig. 1.
Fig. 3 is a local cross-sectional view of an insertion tube 1 when a protection tube 14 is a hard tube.
Fig. 4 is a local cross-sectional view of the insertion tube 1 when the protection tube 14 is a flexible tube.
Fig. 5 is a structure diagram of an insertion end of the insertion tube 1.
Fig. 6 is an overall structure diagram of one embodiment of the present application.

### Element mark number description

- 1: Insertion tube
- 11: Outer tube
- 12: Inner tube
- 13: Bending portion
- 14: Protection tube
- 2: Handle
- 3: Control portion
- 31: Outer ring sleeve
- 32: Inner ring sleeve
- 4: Display portion
- 5: Camera
- 6: Light source
- 7: Instrument tube
- 8: Water tube

### DESCRIPTION OF THE EMBODIMENTS

The implementation modes of the present application will be illustrated by specific embodiments hereinafter, and other advantages and effects of the present application may be easily understood by those skilled in the art from the technical features disclosed in the present application.

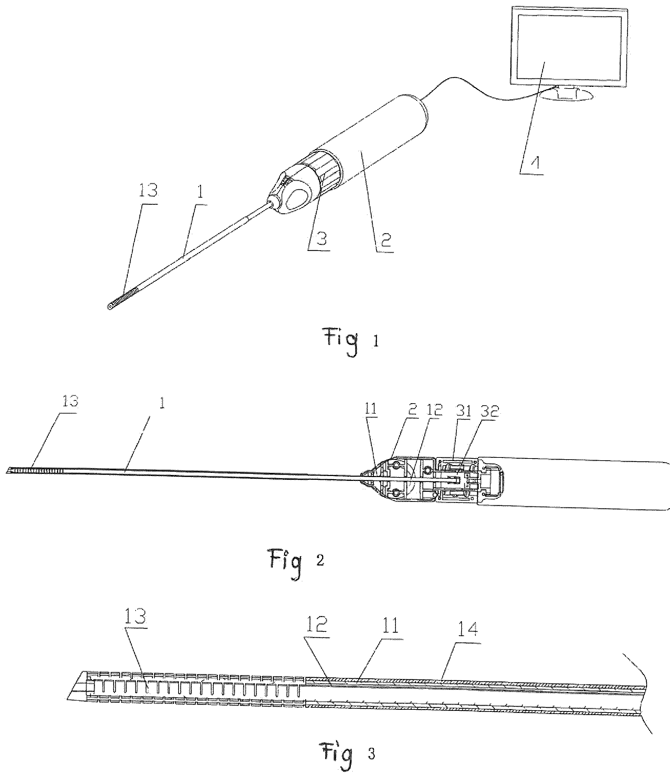

As shown from Fig. 1 to Fig. 5, the present application provides a hard-tube endoscope, comprising an insertion tube 1, a camera 5, a light source 6and a handle 2. The camera 5 and the light source 6 are disposed on an end face of an insertion end of the insertion tube 1, and a non-insertion end of the insertion tube 1 is connected to the handle 2. A display portion 4 is further disposed outside the handle 2, and the display portion 4 is connected with the camera 5 in a wiring or wireless mode. After the insertion tube 1 enters a human body, the camera 5 can perform real-time shooting on the insertion process and transmit shot images to the display portion 4 to display the shot images. When the hard-tube endoscope enters an area, to be observed, in a human body, the camera5 transmits the conditions of the environment in the human body to the display portion 4 in real time for displaying, and a user can inspect a patient according to the images shot by the camera 5so as to find lesions in real time.

Further, the light source 6 can provide a bright shooting environment for the camera 5. In the present application, the light source6 can be a lighting device of any type, such as an LED lamp or an optical fibre illumination device.

Further, in the present application, the display portion 4 can be a display or a displaying device of any specification in any size,a nd the display portion 4 can be connected with the camera 5 through an electric connection wire in a wiring connection mode, and can also be connected with the display through wireless transmitting modules disposed in the handle 2 or in different positions of the hard-tube endoscope in a wireless connection mode. Further, the display portion 4 not only can be independently disposed, but also can be connected with the handle 2 in a pluggable mode or other detachable modes when the display portion 4 is small, so as to be carried conveniently when a user goes out.

In the present application, the insertion tube 1 of the hard-tube endoscope further comprises a bending portion 13. The vision field of the camera 5 can be enlarged through the bending portion 13, so that internal inspection can be performed more comprehensively on a patient.

Further, the bending portion 13 is located at a part, locatable inside a human body, on the insertion tube 1, and is usually disposed at a position near the insertion end of the insertion tube 1. Further, a control portion 3 configured to control the bending portion 13 to be bent is disposed on the handle 2 of the hard-tube endoscope.

Further, in the present application, the control portion 3 comprises an inner ring sleeve 32 and an outer ring sleeve 31. The outer ring sleeve 31is engaged with the inner ring sleeve 32 by means of threads. The outer ring sleeve 31 can rotate around the axis of the handle 2 with respect to the handle 2 only, and the inner ring sleeve 32can axially move with respect to the axis of the handle 2 only. When a user rotates the outer ring sleeve 31, the outer ring sleeve 31 drives the inner ring sleeve 32 to axially and linearly move through the threads, so as to convert rotating movement into linear movement.

In the present application, the insertion tube 1 comprises an elastic inner tube 12 and an elastic outer tube 11 sheathed by each other, and the inner tube 12 and the outer tube 11 are fixedly connected at the position of the insertion end. Due to the fact that the inner tube 12 and the outer tube 11 have certain elasticity, the inner tube 12 and the outer tube 11can recover to original shapes after elastic deformation. A non-insertion end of the inner tube 12 in the handle 2 is fixedly connected to the inner ring sleeve 32, and a non-insertion end of the outer tube 11 is fixedly connected to the handle 2. When the outer ring sleeve 31 is rotated in one direction and the inner ring sleeve32 axially moves along the upstream side of the insertion direction along with the rotation of the outer ring sleeve 31, the inner ring sleeve 32drives the inner tube 12 to axially move along the upstream side of the insertion direction. At the moment, since the inner tube 12 is fixedly connected with one end of the outer tube 11and the inner tube 11 and the handle 2 are static with respect to the inner tube 12, when the inner tube 12 moves towards the insertion direction, the outer tube 11 is ejected towards the insertion direction, resulting in bending deformation of the bending portion 13 of the outer tube 11. As the movement distance of the inner tube 12 increases gradually, the bending degree of the bending portion 13 also increases gradually.

Further, when the outer ring sleeve 31 is rotated reversely, the inner ring sleeve 32 axially moves along the downstream side of the insertion direction along with the rotation of the outer ring sleeve 31, so as to drive the inner tube 12 to axially move along the downstream side of the insertion direction. At the moment, the inner tube 12 returns to an original position gradually, and as the movement distance of the inner tube 12 increases gradually, the bending degree of the bending portion 13 reduces gradually till bending disappears.

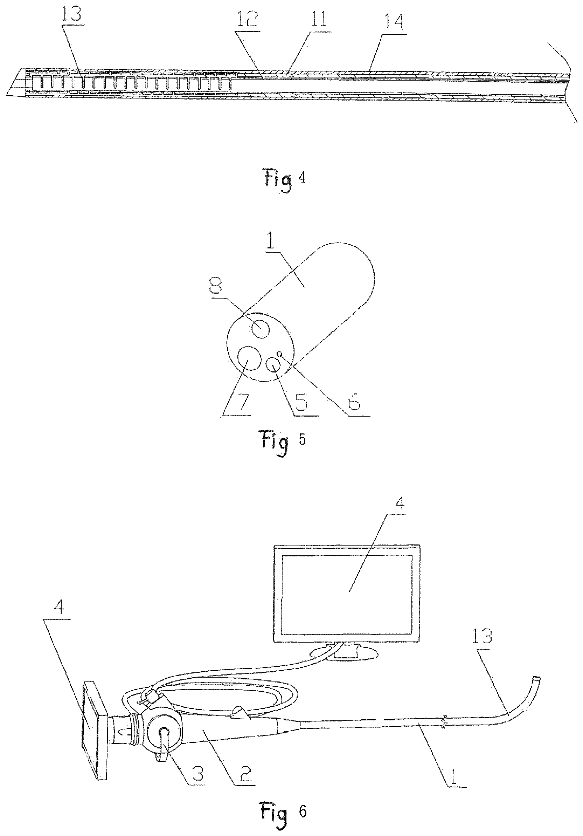

Further, in other embodiments of the present application, as shown in Fig. 6, the control portion 3not only can convert rotating movement into linear movement by means of the inner ring sleeve 32 and the outer ring sleeve 31, so as to make the insertion tube 1 bent, but also can control the inner tube 12 to axially move by means of a control rocker bar or a rotating ring etc., so as to make the bending portion 13 of the insertion tube 1bent and deformed.

In the present application, the bending portion 13 comprises a plurality of annular slots formed in an outer wall face of the outer tube 11 and in an outer wall face of the inner tube 12. In the present application, stainless steel is used to make the inner tube 12 and the outer tube 11 preferably, so that the insertion tube 1of the hard-tube endoscope is harder in texture and bending deformation occurs less easily. When the inner tube 12 moves towards the insertion direction, the inner tube 12ejects the outer tube 11 towards the insertion direction. Since the inner tube 12 and the outer tube 11 are fixedly connected at the position of the insertion end, the inner tube 12suffers from certain resistance during movement towards the insertion direction. In view of this, a plurality of annular slots are formed in a part, needing bending deformation, of the inner tube 12, a plurality of annular slots are also formed in positions, corresponding to the annular slots in the inner tube 12, of the outer tube 11. So that when the inner tube 12 suffers from resistance when moving towards the insertion direction, the inner tube 12 can be bent towards a part with a thinner tube wall, and the outer tube 11 is also bent and deformed along with the inner tube 12, which means bending deformation of the insertion tube 1 is realized at the moment. Besides, due to the fact that the insertion tube 1 provided with the annular slots also has certain elasticity, bending deformation of the insertion tube 1 is reversible, and the insertion tube 1 can be straightened or bent freely according to the requirements of users.

Further, the bending portion 13 is located at a starting site of the insertion end of the insertion tube 1, so that the insertion end provided with the camera 5 can be bent to facilitate observation to a patient. Further, the annular slots are parallel to one another and are perpendicular to the axis of the insertion tube 1. Preferably, the intervals between the annular slots are identical, so that the insertion tube 1can be bent more easily, and the bending effect is better.

Further, the bending range of the bending portion 13 is 0° to 80°, preferably0° to 60°. At the moment, the camera 5 can have the largest vision field and the bending deformation of the insertion tube 1does not fail and can be performed multiple times repeatedly.

In the present application, to better protect the insertion tube 1, the outer tube 11 of the insertion tube 1 is also sheathed by a protection tube 14. One end of the protection tube 14 is fixedly connected with the insertion end of the insertion tube 1, and the other end of the protection tube 14 is disposed in the handle 2 in a floating mode.

Further, the protection tube 14 can be a hard tube with a harder texture or a flexible tube. As shown in Fig. 3, when the protection tube 14is a hard tube, a plurality of annular slots are formed in a position, corresponding to the bending portion 13 of the insertion tube 1, of an outer wall face of the protection tube 14, so that when the insertion tube 1 is bent, the protection tube 14 can be bent along with the bending of the insertion tube 1. As shown in Fig. 4, when the protection tube 14 is a flexible tube, elastic deformation occurs more easily. As a result, bending deformation can be realized even without annular slots in the protection tube 14. When the insertion tube 1 is bent, the protection tube 14 can be bent along with the bending of the insertion tube 1.

In the present application, an instrument tube 7 is arranged in the inner tube 12 of the insertion tube 1. An instrument outlet of the instrument tube 7 is formed in the end face of the insertion end of the insertion tube 1, an instrument inlet of the instrument tube 7 is formed in the handle 2 or on peripheral walls of the insertion tube 1 and the protection tube 14. The instrument tube 7 is a channel for access of a therapeutic instrument. When lesions are found after inspection on a patient, the therapeutic instrument can be inserted through the instrument tube 7 so as to perform treatment. During specific operation, the therapeutic instrument is inserted through the instrument inlet, penetrates through the instrument pipe 7 and then stretch out through the instrument outlet for treatment or sampling for the patient.

Further, a therapeutic instrument can be disposed in the instrument tube 7 in a telescopic mode. When a patient needs to be treated, the therapeutic instrument is made to stretch out from the instrument tube. When treatment is finished or not required, the therapeutic instrument is locked in the instrument tube 7 to avoid injury to the patient.

Further, in the present application, a water tube 8 is disposed in the insertion tube 1 preferably. A water outlet of the water tube 8 is formed in the end face of the insertion end of the insertion tube 1, and a water inlet of the water tube 8 is formed in the handle 2 or on peripheral walls of the insertion tube 1 and the protection tube 14. In the process of making the insertion tube 1 enter the body of a patient, the insertion tube 1 might encounter certain obstacles or bloody water which can block the camera 5 to further affect the shooting effect, and normal saline can be injected into the water tube 8 at the moment to wash away the obstacles or dilute the bloody water, so as to clear the way for the camera 5. Further, a liquid absorption device can be disposed at the position of the water inlet of the water tube 8. After the shooting environment of the camera 5 is cleared, injected normal saline can be absorbed by the liquid absorption device, so that the shooting environment is clearer, and harmful effects on a patient caused by liquid left in the body are avoided at the same time.

Based on the above, the hard-tube endoscope according to the present application has a wide visibility range, can perform comprehensive internal inspection on a patient as needed, can also treat found lesions in time to reduce harm to a patient to be minimum, and is simple in structure, convenient to use and worthy of wide application and popularization.

The above embodiments are only for exemplary explanation of the principles and effects of the present application.

## Claims

1. A hard-tube endoscope, comprising an insertion tube (1), a camera (5), a light source (6) and a handle (2), the camera (5) and the light source (6) being disposed on an end face of an insertion end of the insertion tube (1), and a non-insertion end of the insertion tube (1) being connected to the handle (2); wherein the insertion tube (1) further comprises a bending portion (13), the bending portion (13) is located at a part, locatable inside a human body, on the insertion tube (1), and a control portion (3) configured to control the bending portion (13) to be bent is disposed on the handle (2); **characterized in that**
the control portion (3) comprises an inner ring sleeve (32) and an outer ring sleeve (31), the outer ring sleeve (31) is engaged with the inner ring sleeve (32) by means of threads, the outer ring sleeve (31) can rotate only around the axis of the handle (2) with respect to the handle (2), and the inner ring sleeve (32) can axially move only with respect to the axis of the handle (2);
the insertion tube (1) comprises an elastic inner tube (12) and an elastic outer tube (11) sheathed by each other, and the inner tube (12) and the outer tube (11) are fixedly connected at the position of the insertion end; a non-insertion end of the inner tube (12) in the handle (2) is fixedly connected to the inner ring sleeve (32), and a non-insertion end of the outer tube (11) is fixedly connected to the handle (2);
when the outer ring sleeve (31) is rotated and the inner ring sleeve (32) axially moves along the upstream side of the insertion direction along with the rotation of the outer ring sleeve (31), the inner ring sleeve (32) drives the inner tube (12) to axially move along the upstream side of the insertion direction, and as the movement distance of the inner tube (12) increases gradually, the bending degree of the bending portion (13) also increases gradually;
when the outer ring sleeve (31) is rotated reversely, the inner ring sleeve (32) axially moves along the downstream side of the insertion direction along with the rotation of the outer ring sleeve (31), so as to drive the inner tube (12) to axially move along the downstream side of the insertion direction, and as the movement distance of the inner tube (12) increases gradually, the bending degree of the bending portion (13) reduces gradually till bending disappears.

2. The hard-tube endoscope according to claim 1, **characterized in that** the bending range of the bending portion (13) is 0° to 80°.

3. The hard-tube endoscope according to claim 2, **characterized in that** the bending portion (13) comprises a plurality of annular slots formed on outer wall faces of the outer tube (11) and the inner tube (12).

4. The hard-tube endoscope according to claim 3, **characterized in that** the bending portion (13) is further located at the starting site of the insertion end of the insertion tube (1).

5. The hard-tube endoscope according to claim 4, **characterized in that** the annular slots are parallel to one another, and are perpendicular to the axis of the insertion tube (1).

6. The hard-tube endoscope according to any one of claims 1 to 5, **characterized in that** the outer tube (11) is further sheathed by a protection tube (14), one end of the protection tube (14) is fixedly connected with the insertion end of the insertion tube (1), and the other end of the protection tube (14) is disposed in the handle (2) in a floating mode.

7. The hard-tube endoscope according to claim 6, **characterized in that** the protection tube (14) is a hard tube, and a plurality of annular slots are also formed in a position, corresponding to the bending portion (13) of the insertion tube (1), of an outer wall face of the protection tube (14).

8. The hard-tube endoscope according to claim 6, **characterized in that** the protection tube (14) is a flexible tube.

9. The hard-tube endoscope according to claim 6, **characterized in that** an instrument tube (7) is disposed in the inner tube (12) of the insertion tube (1), an instrument outlet of the instrument tube (7) is formed in the end face of the insertion end of the insertion tube (1), and an instrument inlet of the instrument tube is formed in the handle (2) or on peripheral walls of the insertion tube (1) and the protection tube (14).

10. The hard-tube endoscope according to claim 9, **characterized in that** a water tube (8) is disposed in the insertion tube (1), a water outlet of the water tube (8) is formed in the end face of the insertion end of the insertion tube (1), and a water inlet of the water tube (8) is formed in the handle (2) or on peripheral walls of the insertion tube (1) and the protection tube (14).

11. The hard-tube endoscope according to claim 5, **characterized in that** the annular slots formed on outer wall faces of the outer tube (11) are formed at positions corresponding to the annular slots in the inner tube (12), so that when moving towards the insertion direction, the inner tube (12) is bent towards a part having a thinner tube wall, and the outer tube (11) is also bent and deformed along with the inner tube (12).

12. The hard-tube endoscope according to claim 11, **characterized in that** the inner tube (12) and the outer tube (11) are made of stainless steel.

## Patentansprüche

1. Hartrohr-Endoskop, das ein Einführungsrohr (2), eine Kamera (5), eine Lichtquelle (6) und einen Griff (2) aufweist, wobei die Kamera (5) und die Lichtquelle (6) an einer Endfläche eines Einführungsendes des Einführungsrohrs (1) angeordnet sind und ein Nicht-Einführungsende des Einführungsrohrs (1) mit dem Griff (2) verbunden ist, wobei
das Einführungsrohr (1) ferner einen Biegeabschnitt (13) aufweist, der Biegeabschnitt (13) an einem Teil, das im Inneren eines menschlichen Körpers anordenbar ist, auf dem Einführungsrohr (1) angeordnet ist, und ein Steuerabschnitt (3), der konfiguriert ist, um den zu biegenden Biegeabschnitt (13) zu steuern, auf dem Griff (2) angeordnet ist,
**dadurch gekennzeichnet, dass**
der Steuerabschnitt (3) eine innere Ringhülse (32) und eine äußere Ringhülse (31) aufweist, die äußere Ringhülse (31) mit der inneren Ringhülse (32) mittels Gewinde in Eingriff steht, die äußere Ringhülse (31) nur um die Achse des Handgriffs (2) in Bezug auf den Handgriff (2) drehbar ist und die innere Ringhülse (32) nur in Bezug auf die Achse des Handgriffs (2) axial beweglich ist;
das Einführungsrohr (1) ein elastisches inneres Rohr (12) und ein elastisches äußeres Rohr (11) umfasst, die miteinander ummantelt sind, und das innere Rohr (12) und das äußere Rohr (11) an der Position des Einführungsendes fest verbunden sind; ein Nicht-Einführungsende des inneren Rohres (12) in den Griff (2) fest mit der inneren Ringhülse (32) verbunden ist, und ein Nicht-Einführungsende des äußeren Rohres (11) fest mit dem Griff (2) verbunden ist;
wobei, wenn die äußere Ringhülse (31) gedreht wird und die innere Ringhülse (32) sich axial entlang der stromaufwärtigen Seite der Einführungsrichtung zusammen mit der Drehung der äußeren Ringhülse (31) bewegt, die innere Ringhülse (32) das innere Rohr (12) antreibt, um sich axial entlang der stromaufwärtigen Seite der Einführungsrichtung zu bewegen, und wobei, wenn der Bewegungsabstand des inneren Rohrs (12) allmählich zunimmt, auch der Biegungsgrad des Biegeabschnitts (13) allmählich zunimmt;
wobei, wenn die äußere Ringhülse (31) zurück gedreht wird, sich die innere Ringhülse (32) axial entlang der stromabwärtigen Seite der Einführungsrichtung zusammen mit der Drehung der äußeren Ringhülse (31) bewegt, um das innere Rohr (12) so anzutreiben, dass es sich axial entlang der stromabwärtigen Seite der Einführungsrichtung bewegt, und wobei, wenn die Bewegungsstrecke des inneren Rohrs (12) allmählich zunimmt, sich der Biegungsgrad des Biegeabschnitts (13) allmählich verringert, bis die Biegung verschwindet.

2. Hartrohr-Endoskop nach Anspruch 1, **dadurch gekennzeichnet, dass** der Biegebereich des Biegeabschnitts (13) 0° bis 80° beträgt.

3. Hartrohr-Endoskop nach Anspruch 2, **dadurch gekennzeichnet, dass** der Biegeabschnitt (13) eine Vielzahl von ringförmigen Schlitzen aufweist, die an den Außenwandflächen des Außenrohrs (11) und des Innenrohrs (12) ausgebildet sind.

4. Hartrohr-Endoskop nach Anspruch 3, **dadurch gekennzeichnet, dass** sich der Biegeabschnitt (13) weiter an der Anfangsstelle des Einführungsendes des Einführungsrohres (1) befindet.

5. Hartrohr-Endoskop nach Anspruch 4, **dadurch gekennzeichnet, dass** die ringförmigen Schlitze parallel zueinander und senkrecht zur Achse des Einführungsrohrs (1) verlaufen.

6. Hartrohr-Endoskop nach den Ansprüchen 1-5, **dadurch gekennzeichnet, dass** das Außenrohr (11) ferner von einem Schutzrohr (14) umhüllt ist, ein Ende des Schutzrohres (13) fest mit dem Einführungsende des Einführungsrohres (1) verbunden ist und das andere Ende des Schutzrohres (14) schwimmend im Handgriff (2) angeordnet ist.

7. Hartrohr-Endoskop nach Anspruch 6, **dadurch gekennzeichnet, daß** das Schutzrohr (14) ein hartes Rohr ist, und eine Vielzahl von ringförmigen Schlitzen auch in einer dem Biegeabschnitt (13) des Einführungsrohres (1) entsprechenden Position einer äußeren Wandfläche des Schutzrohres (14) ausgebildet ist.

8. Hartrohr-Endoskop nach Anspruch 6, **dadurch gekennzeichnet, dass** der Schutzschlauch (14) ein flexibler Schlauch ist.

9. Hartrohr-Endoskop nach Anspruch 6, **dadurch gekennzeichnet, dass** ein Instrumentenrohr (7) im Innenrohr (12) des Einführungsrohrs (1) angeordnet ist, ein Instrumentenauslass des Instrumentenrohrs (7) in der Endfläche des Einführungsendes des Einführungsrohrs (1) ausgebildet ist und ein Instrumenteneinlass des Instrumentenrohrs im Griff (2) oder an Umfangswänden des Einführungsrohrs (1) und des Schutzrohrs (14) ausgebildet ist.

10. Hartrohr-Endoskop nach Anspruch 9, **dadurch gekennzeichnet, dass** im Einführungsrohr (1) ein Wasserrohr (8) angeordnet ist, dass ein Wasserauslass des Wasserrohres (8) in der Endfläche des Einführungsendes des Einführungsrohres (1) ausgebildet ist, und dass ein Wassereinlass des Wasserrohres (8) im Handgriff (2) oder an Umfangswänden des Einführungsrohres (1) und des Schutzrohres (14) ausgebildet ist.

11. Hartrohr-Endoskop nach Anspruch 5, **dadurch gekennzeichnet, dass** die an den Außenwandflächen des Außenrohrs (11) ausgebildeten ringförmigen Schlitze an Positionen ausgebildet sind, die den ringförmigen Schlitzen im Innenrohr (12) entsprechen, so dass bei einer Bewegung in Einführungsrichtung das Innenrohr (12) zu einem Teil mit einer dünneren Rohrwand hin gebogen wird und das Außenrohr (11) zusammen mit dem Innenrohr (12) ebenfalls gebogen und verformt wird.

12. Hartrohr-Endoskop nach Anspruch 11, **dadurch gekennzeichnet, dass** das Innenrohr (12) und das Außenrohr (11) aus rostfreiem Stahl hergestellt sind.

## Revendications

1. Un endoscope à tube rigide, comprenant un tube d'insertion (1), une caméra (5), une source lumineuse (6) et une poignée (2), la caméra (5) et la source lumineuse (6) étant disposés sur une face d'extrémité d'une extrémité d'insertion du tube d'insertion (1), et une extrémité de non-insertion du tube d'insertion (1) étant connectée à la poignée (2) ; étant précisé que le tube d'insertion (1) comprend en outre une section flexible (13), la section flexible (13) est située à un point localisable à l'intérieur d'un corps humain, sur le tube d'insertion (1), et une section de contrôle (3) configurée pour contrôler la section flexible (13) prévue pour être courbée est disposée sur la poignée (2) ;
**caractérisé en ce que** :
la section de contrôle (3) comprend une gaine annulaire interne (32) et une gaine annulaire externe (31), la gaine annulaire externe (31) est en prise avec la gaine annulaire interne (32) au moyen de fils, la gaine annulaire externe (31) ne peut tourner qu'autour de l'axe de la poignée (2) par rapport à la poignée (2), et la gaine annulaire interne (32) ne peut bouger axialement que par rapport à l'axe de la poignée (2) ;
le tube d'insertion (1) comprend un tube interne élastique (12) et un tube externe élastique (11), gainés l'un par l'autre, et le tube interne (12) et le tube externe (11) sont connectée de manière fixe au point de l'extrémité d'insertion ; une extrémité de non-insertion du tube interne (12) dans la poignée (2) est connectée de manière fixe à la gaine annulaire interne (32), et une extrémité de non-insertion du tube externe (11) est connectée de manière fixe à la poignée (2) ;
lorsque la gaine annulaire externe (31) est tournée et que la gaine annulaire interne (32) se déplace axialement le long du coté amont de la direction d'insertion en même temps que la rotation de la gaine annulaire externe (31), la gaine annulaire interne (32) pousse le tube interne (12) à se déplacer axialement le long du côté amont de la direction d'insertion, et tandis que la distance de mouvement du tube interne (12) augmente graduellement, le degré de courbure de la section flexible (13) augmente aussi graduellement ;
lorsque la gaine annulaire externe (31) est tournée dans le sens inverse, la gaine annulaire interne (32) se déplace axialement le long du coté aval de la direction d'insertion en même temps que la rotation de la gaine annulaire externe (31), de sorte à pousser le tube interne (12) à se déplacer axialement le long du côté aval de la direction d'insertion, et tandis que la distance de mouvement du tube interne (12) augmente graduellement, le degré de courbure de la section flexible (13) diminue graduellement jusqu'à ce que la courbure disparaisse.

2. L'endoscope à tube rigide selon la Revendication 1, **caractérisé en ce que** la plage de flexion de la section flexible (13) s'étend de 0° à 80°.

3. L'endoscope à tube rigide selon la Revendication 2, **caractérisé en ce que** la section flexible (13) comprend une pluralité de fentes annulaires formées sur les faces de la paroi externe du tube externe (11) et du tube interne (12).

4. L'endoscope à tube rigide selon la Revendication 3, **caractérisé en ce que** la section flexible (13) est en outre localisée au début de l'extrémité d'insertion du tube d'insertion (1).

5. L'endoscope à tube rigide selon la Revendication 4, **caractérisé en ce que** les fentes annulaires sont parallèles les unes aux autres, et perpendiculaires à l'axe du tube d'insertion (1).

6. L'endoscope à tube rigide selon l'une quelconque des Revendications 1 à 5, **caractérisé en ce que** le tube externe (11) est en outre gainé par un tube de protection (14), une extrémité du tube de protection (14) est connectée de manière fixe à l'extrémité d'insertion du tube d'insertion (1), et l'autre extrémité du tube de protection (14) est placée dans la poignée (2) dans un mode flottant.

7. L'endoscope à tube rigide selon la Revendication 6, **caractérisé en ce que** le tube de protection (14) est un tube rigide, et une pluralité de fentes annulaires sont également formées sur la section flexible (13) du tube d'insertion (1), d'une face de paroi externe du tube de protection (14).

8. L'endoscope à tube rigide selon la Revendication 6, **caractérisé en ce que** le tube de protection (14) est un tube flexible.

9. L'endoscope à tube rigide selon la Revendication 6, **caractérisé en ce qu'**un tube d'instrument (7) est logé dans le tube interne (12) du tube d'insertion (1), une sortie d'instrument du tube d'instrument (7) est prévue dans la face d'extrémité de l'extrémité d'insertion du tube d'insertion (1), et une entrée d'instrument du tube d'instrument est prévue dans la poignée (2) ou sur des parois périphériques du tube d'insertion (1) et du tube de protection (14).

10. L'endoscope à tube rigide selon la Revendication 9, **caractérisé en ce qu'**un tube d'eau (8) est logé dans le tube d'insertion (1), une sortie d'eau du tube d'eau (8) est prévue dans la face d'extrémité de l'extrémité d'insertion du tube d'insertion (1), et une entrée d'eau du tube d'eau (8) est prévue dans la poignée (2) ou sur des parois périphériques du tube d'insertion (1) et du tube de protection (14).

11. L'endoscope à tube rigide selon la Revendication 5, **caractérisé en ce que** les fentes annulaires formées sur les faces externes du tube externe (11) sont prévues à des positions correspondant aux fentes annulaires dans le tube interne (12), de sorte que lorsqu'il se déplace dans la direction d'insertion, le tube interne (12) est courbé vers une partie présentant une paroi de tube plus fine, et le tube externe (11) est également courbé et déformé avec le tube interne (12).

12. L'endoscope à tube rigide selon la Revendication 11, **caractérisé en ce que** le tube interne (12) et le tube externe (11) sont en acier inoxydable.
